# EUROPEAN PATENT SPECIFICATION

(11) **EP 0 974 343 B1**
(45) Date of publication and mention of the grant of the patent: **29.09.2004**
(21) Application number: 99202373.9
(22) Date of filing: 19.07.1999
(51) Int. Cl.: A61K 9/20, A61K 31/165

(54) **Process for manufacturing a solid metoprolol composition**
Verfahren zur Herstellung einer festen Zubereitung enthaltend Metoprolol
Procédé de préparation d'une composition solide contentant du metoprolol

(30) Priority: 22.07.1998 US 120914; 04.09.1998 EP 98402192
(43) Date of publication of application: 26.01.2000
(73) Proprietor: Pharma Pass II LLC, Irvine, CA 92618 (US)
(72) Inventor: Seth, Pawan, Irvine, CA 92612 (US)
(74) Representative: Pochart, François

(56) References cited:
- WO-A-96/16638
- WO-A-96/32097
- WO-A-97/18814
- US-A- 4 343 789

## Description

The present invention relates to a process. For the preparation of a solid metoprolol composition, containing polyethylene oxide and metoprolol.

Certain medicaments need to be formulated in so-called delayed-release or sustained release form.

Polyethylene oxide referred to as PEO below is moreover known as a component of medicaments in tablet form designed to be administered by oral route. This compound is marketed by the Union Carbide Corporation under the commercial name Polyox. The use of PEO for formulating medicaments has furthermore been the subject matter of many earlier patents.

EP-A-0 277 092, in the name of Ciba-Geigy relates to a composition comprising a casing in a material that is porous to water but not to the active ingredient, surrounding a central core consisting of a mixture of a substance that is weakly soluble in water, and a hydrophilic swelling material, said material consisting of a mixture of PEO and a vinyl pyrrolidone/vinyl acetate polymer. The composition in that patent is an example of current compositions in which a core which swells when exposed to water is surrounded by a water-porous material, release of the active ingredient being delayed or sustained as a result of the time necessary to expand the core, and for diffusion to take place through the casing following the penetration of water.

WO A 96 32097 and WO A 97 18814 disclose a process for preparing a pharmaceutical composition wherein the active ingredient, the PEO and additive(s) are mixed in a dry state.

The abstract of the US-A-4,404,183 and US-A-4, 343,789 discloses two embodiments of a sustained release composition. In the first embodiment, the composition contains PEO, the active ingredient in an amorphous form, and a basic component. In the second embodiment, the active ingredient is nicardipine in an amorphous state, it being possible to omit the basic component.

Actually, the compositions according to the prior art are complex, require specific active ingredients or are provided in a specific form. Moreover, the results achieved are not always very good.

The composition which is prepared according to the process of the present invention, comprises metoprolol, and has a remarkable delaying or sustaining effect.

Thus, the present invention provides a process for the preparation of a solid composition comprising, by weight based on the total weight of the composition:
(a) from 1 to 70% of metoprolol which is not in an amorphous form;
(b) from 10 to 95% of polyethylene oxide;
(c) the balance consisting of conventional additives; comprising the steps of :
   mixing said metoprolol active ingredient and optionally one or several additives with an aqueous solution for a sufficient period of time; then
   adolina the PEO and mixing for a sufficient periode of time.

The expression "solid composition" means that the composition is in a tablet or mini-tablet form, these in their turn being able to be encapsulated using for example the conventional hard gelatin.

Metoprolol active ingredient is intended to cover especially the salts of metoprolol, such as succinate and tartrate. Metoprolol tartrate is the preferred active ingredient.

The expression "not in amorphous form" should be understood in its conventional meaning. Various sources give a definition of this term "amorphous" as meaning non-crystalline, lacking the lattice structure characterizing the crystalline state. The following references, which provide a definition of the term amorphous (or the opposite thereof) are, in a non-limiting manner: Hawley's, Condensed Chemical Dictionary, 12th Edition, p. 71; Handbook of Chemistry and Physics, 65th Edition, F-67; The Theory and Practice of Industrial Pharmacy, 1970, pp. 253-255; Remington's Pharmaceutical Sciences, 14th Edition, p. 182; General Chemistry 1992, pp.314-315; Encyclopedia of Pharmaceutical Technology, vol I, pp. 12-13.

The expression "excluding basic compounds" should be understood as excluding the presence of a compound or a group of compounds that confer a basic nature on the composition, in other words a pH greater than 7, when the composition is diluted in water at a rate of 10g per liter of water. In particular, this term should be taken as excluding the presence of one or several basic component(s) such as described in column 1, lines 38 to 62 of US-A-4,404,183 if no acid compound is counteracting the effect of said basic compound, or if the basic compound is present in a relatively large amount.

According to one preferred embodiment, the composition prepared according to the invention, comprises:
(a) from 5 to 45% of metoprolol active ingredient;
(b) from 25 to 70% polyethylene oxide
(c) the balance consisting of conventional additives, excluding basic components.

According to one alternative embodiment, in the composition prepared according to the invention, the polyethylene oxide has a molecular weight which varies from 50,000 to 8,000,000, and preferably from 100,000 to 3,000,000. The required molecular weight for the PEO can be obtained by mixing PEO of differing molecular weights, that are available commercially.

According to a further embodiment, in the composition prepared according to the invention, the balance consisting of conventional additives comprises microcrystalline cellulose, lactose, ascorbic acid, pigments, plasticizing agents, lubricants and so on. Obviously, other conventional additives known to those skilled in the art can be employed.

According to one embodiment of the invention, in a composition, the balance consisting of conventional additives comprises magnesium stearate and/or glycerol behenate and/or sodium stearyl fumarate, which is employed as a lubricant ensuring better compression of the composition when provided in tablet form, for example.

According to one or alternative embodiment, the composition is additionally coated. Surface coating is employed for the purposes of improving appearance making the drug more readily acceptable to the patient, or for dimensionally stabilizing the compressed tablet. The coating can be a conventional coating suitable for enteral use. It is obtained using any conventional technique employing conventional ingredients. A surface coating can for example be obtained using a quick-dissolving film. It should be noted that the coating according to this invention is fundamentally different from the coating used in EP-A-0,277,092 as one does not encounter, in the invention, the dichotomy (water-swellable core)/(water-porous coating), and moreover, the coating in the invention dissolves and/or disintegrates whereas the coating in EP-A-0,277,092 does not dissolve.

The present solid compositions are suitable for the administration of medicaments.

A process of the prior art comprises the steps of:
(i) mixing in the dry state and for a sufficient time, the metoprolol active ingredient, polyethylene oxide and optionally, one or several additives;
(ii) optionally adding solvent when this is used, followed by mixing for a sufficient period of time;
(iii) granulation;
(iv) drying the granules thus formed for a sufficient period of time;
(v) optionally adding one of more additives, with mixing in the dry state for a sufficient time and passage through a suitable sieve;
(vi) optionally adding one or several additives and mixing in the dry state for a sufficient period of time;
(vii) compressing the mixture obtained from the preceding steps to obtain the desired compressed tablet; and
(viii) optionally coating said compressed tablet.

The solvent employed, when use is made of a solvent, is preferably an alcohol. The solvent is eliminated by drying at one point or another in the process, and is substantially not encountered in the final composition.

Granulation can be performed by passage through a suitable sieve.

The drying step can be implemented e.g. by using a fluidized bed or a drying oven.

The above granulation and drying steps can be combined by using a fluidized bed granulator (e.g. with a top spray chamber).

The choice of mixing times, apparatus used, sieve mesh, and other operating conditions are within the province of the normal knowledge of those skilled in the art.

Without wishing to be bound by any theory, the applicant believes that the PEO, in the formulation, forms a hydrogel from contact with water. This hydrogel dissolves more or less rapidly as a function of the molecular weight of the PEO employed. Choosing the molecular weight of the PEO, in combination with a suitable choice of the weight concentrations of the active ingredient, of PEO, and of additives enables release of the active ingredient to be controlled.

The carrying out of the process as depicted above with water as the wetting liquid is difficult. Indeed, PEO is a water-swellable polymer which instantaneously forms agglomerates when it comes into contact with the droplets of water. This leads to the final formation of elastic lumps within the heart of the mass to be granulated, these being difficult to size, notably on an oscillating granulation grid.

The instant improved process is thus a method for wet granulation of PEO, using water as the wetting liquid. It is now possible to avoid both the problems linked to direct compression (the heterogeneous nature of the mixture) as well as problems associated with organic solvents (cost, inflammability, and toxicity towards the operator and the environment).

The improved process consists in a two-stage wetting allowing PEO to be hydrated without forming lumps. During the first wetting step, aqueous wetting of the active ingredient alone is carried out or, if appropriate, of the active ingredient and one or several excipients such as, for example, microcrystalline cellulose or lactose. During the second wetting step, the PEO is added to the foregoing wet mixture. These two wetting steps are both simply referred to as the "wetting step" herein.

The wetting liquid can if necessary include substances in solution in order to modify the properties of the granulated product, and to improve the wetting operation. Thus, binders such as hydroxypropylmethyl cellulose or polyvinylpyrrolidone can be employed in order to improve the properties of the granulated product. When such substances are used, their concentration can vary up to about 20%, for example between 1 and 10%.

It is important to note that in all cases, water constitutes substantially the only solvent of the wetting liquid in the improved process.

The wetting operation can be carried out in any conventional blender described in the literature such as for example apparatus manufactured by Vector, Moritz, Collette, Bouvard, Lodige, etc. Such blenders can employ kneading, blades, planetary arrangements or, preferably but not obligatorily, high speed with or without a lump-breaking knife. Wetting takes place for a period comprised between 0,5 and 15 min, for example between 2 and 6 min.

Preferably, but not indispensably, slight "over-wetting" of the powders is done which renders the following operations easier. "Over-wetting" should be taken to mean "adding liquid in excess to the amount required to agglomerate the powder mixture".

The excess water added during the possible slight over-wetting in the first step improves agglomeration of the PEO to the active ingredient and, if present, to the other excipients present.

For example, the weight ratios water/composition or water/PEO may vary respectively from 0.13 to 0.36 and 0.18 to 0.5.

Once the first wetting step has been performed, the PEO is added into the apparatus and a supplementary mixing is performed thereby achieving supplementary wetting of the PEO. During this second mixing, the PEO gets integrated into the granulated product and swells slightly. However, surprisingly, there is no formation of lumps or clods, contrary to what is obtained in conventional granulation, using one single step, as described in the literature.

This last operation can be carried out in the same mixer as was used for the first step, or any other conventional blender as described above. The mixing time is comprised between 0,5 and 15 min, for example between 2 and 6 min.

The process includes other steps which are conventional in the art.

One prior step in which the active ingredient is intimately mixed with the additives, when present, can be provided.

A granulation step notably is carried out after incorporation and mixing of the PEO. This granulation step can be done on a screen having a suitable mesh size.

A drying step, notably using a fluidized bed or a drying oven, is also implemented, notably following the granulation step. The drying step is considered as having been carried out when the residual water content is below 10%, for example below 3%, depending on the kind and amount of ingredients.

The above granulation and drying steps can be combined by using a fluidized bed granulator (e.g. with a top spray chamber).

Steps in which additives are added and blended can be included, if necessary.

A step in which the granules, notably dry granules, are size-graded can be provided. Size grading is carried out also on a screen having a mesh size comprised for example between 100 and 1000 µm.

Final compression steps and, optionally, tablet coating steps are also provided. Notably, a step in which a compression additive is added can be provided after drying, but prior to compression. These final compression steps are obviously not necessary for preparing capsules enclosing the granules.

It is clear that the above order for the steps, apart from that of wetting, is not fixed; one could for example add the compression additives right at the beginning or immediately following addition of the PEO. Similarly, a granulation step could optionally be carried out before adding the PEO.

Choice of the duration of mixing, the apparatus, the screens and other operating conditions is a matter for the normal skill and judgement of the person skilled in the art.

The improved process is thus a process for preparing a solid pharmaceutical composition comprising, by weight based on the total weight of the composition:
(a) from 1 to 70% of active ingredient which is not in an amorphous form;
(b) from 10 to 95% of polyethylene oxide;
(c) the balance consisting of conventional additives, comprising the steps of:
   (i) mixing said active ingredient and optionally one or several additives with an aqueous solution for a sufficient period of time; then
   (ii) adding the PEO, and mixing for a sufficient period of time.

According to one embodiment, step (i) of mixing with the aqueous solution is carried out in the presence of excess liquid.

According to another embodiment, said aqueous solution consists of water, and, optionally, water-soluble additives.

According to yet another embodiment, the improved process further comprises a granulation step; said granulation step can be carried out on a screen of suitable mesh size or using a fluidized bed granulator.

According to yet another embodiment, the improved process additionally comprises a drying step.

According to yet another embodiment, the improved process additionally comprises a compression step; said compression step can be preceded by a step in which additives are added and blended.

A specific embodiment of the instant improved process is a process comprising the steps of:
(i) mixing, for a sufficient period of time, active ingredient and, optionally, one of several additives, with said aqueous solution; then
(ii) adding PEO and mixing for a sufficient period of time;
(iii) granulating the mixture obtained from step (ii);
(iv) drying granules thus formed for a sufficient period of time;
(v) optionally, adding one or several additives, and mixing in the dry state for a sufficient period of time;
(vi) compressing the mixture obtained from the preceding step into a tablet.

Preferably, step (i) of mixing with the aqueous solution is carried out in the presence of excess liquid.

Preferably steps (iii) and (iv) are carried out as a single step in a fluidized bed granulator.

Another specific embodiment of the invention corresponds to the improved process where granulation (and drying) steps are carried out before the PEO is added. Mixing, granulating (and drying) are preferably carried out at once, e.g. in a fluidized bed granulator.

Said alternate specific embodiment of the instant improved process is a process comprising the steps of:
(i) mixing, for a sufficient period of time, active ingredient and, optionally, one of several additives, with said aqueous solution; then
(ii) granulating the mixture obtained from step (i);
(iii) optionally drying the granules thus formed for a sufficient period of time;
(iv) adding PEO and optionally one or several additives, and mixing for a sufficient period of time;
(v) compressing the mixture obtained from the preceding step into a tablet.

Preferably, steps (i) to (iii) are carried out as a single step in a fluidized bed granulator.

Preferably, the aqueous solution comprises water and a binder in an amount up to 20%.

The examples below are provided as examples illustrating the invention and should not be considered as limiting its scope. In the examples, although the amount water employed is given, it should be understood that the water is substantially absent in the final composition. In the examples, tablet hardness is measured using a Schleuniger 4M hardness tester.

### Example 1:

The following formulation was prepared uncoated tablet (in mg)

| | |
|---|---|
| Metoprolol salt | 200 |
| Polyox® WSR N 301 (MW=4000000) | 300 |
| Avicel® PH 101 | 54 |
| Plasdone® K29-32 | 8 |
| Purified water | 60 |
| Aerosil® 200 | 3 |
| Sodium stearyl fumarate | 5 |

The manner of preparation was as follows. Metoprolol and Avicel® were introduced into a Stephan® UMC5 blender and blending was carried out for 5 minutes. The Plasdone® K29-32 was dissolved in water, and the powder was wetted with this solution. Blending was continued for 5 minutes. Next, the Polyox® was added and blending was continued for a further 5 minutes Granulation was performed on an oscillating Erweka® FGS granulator provided with a 1.6 mm mesh grid. The product was dried on a fluidized bed down to a constant humidity. Size-grading was carried out on an oscillating granulator with a 0.8 mm mesh grid. The Aerosil® 200, any lumps previously being broken up, and the sodium stearyl fumarate were added. Blending was continued for 5 minutes. Next, the powder was compressed on a rotary Fette® P2 press provided with 12 mm diameter and 12 mm radius of curvature punches. The nominal mass of the tablet was 570 mg and its average hardness was set to 18 kg. These tablets were then given a film coating using a conventional formulation (e.g. Opadry®.

### Example 2:

The following formulation was prepared uncoated tablet (in mg)

| | |
|---|---|
| Metoprolol tartrate | 200 |
| Polyox® coagulant | 303 |
| Avicel® PH 302 | 100 |
| Plasdone® K29-32 | 16 |
| Aerosil® 200 | 2 |
| Sodium stearyl fumarate | 9 |
| Purified water | 200 |

The manner of preparation was as follows. Metoprolol tartrate, Avicel® and Aerosil® were introduced into a Stephan UMC5 blender and blending was carried out for 5 minutes. The Plasdone® K29-32 was dissolved in water. The powder mixture was granulated with the solution in a fluidized bed granulator (with a top spray chamber), using the following spraying parameters:
Air flow (m³/h) 65 m³/h
Liquid flow (g/min) 16 g/min
Inlet temperature 65°C
Spraying pressure 2.0 bar

Next, the Polyox® and the sodium stearyl fumarate were added and blending was continued for a further 5 minutes. Next, the powder was compressed on a rotary Fette® P2 press provided with 12 mm diameter and 12 mm radius of curvature punches. These tablets were then given a film coating using a conventional formulation (e.g. Opadry).

## Claims

1. Process for preparing a solid composition comprising, by weight based on the total weight of the composition:
(a) from 1 to 70% of metoprolol active ingredient which is not in an amorphous form;
(b) from 10 to 95% of polyethylene oxide (PEO);
(c) the balance consisting of conventional additives;
comprising the steps of:
(i) mixing said metoprolol active ingredient and optionally one or several additives with an aqueous solution for a sufficient period of time; then
(ii) adding the PEO, and mixing for a sufficient period of time.

2. The process according to claim 1, wherein the metoprolol active ingredient is metoprolol tartrate.

3. The process according to claim 1 or 2, wherein the said composition comprises :
(a) from 5 to 45% of metoprolol active ingredient which is not in an amorphous form ;
(b) from 20 to 70% of polyethylene oxide;
(c) the balance consisting of conventional additives, excluding basic components.

4. The process according to any one of claim 1 to 3, wherein the polyethylene oxide has a molecular weight which varies from 50,000 to 8,000,000, and preferably from 300,000 to 3,000,000.

5. The process according anyone of claim 1 to 4, wherein the balance consisting of conventional additives comprises microcrystalline cellulose, lactose, ascorbic acid, pigments, plasticizing agents, lubricants.

6. The process according to anyone of claim 1 to 5, in which step (i) of mixing with the aqueous solution is carried out in the presence of excess liquid.

7. The process according to anyone of claim 1 to 6, further comprising a granulation step.

8. The process according to claim 7, in which said granulation step is carried out on a screen of suitable mesh size.

9. The process according to claim 7, in which said granulation step is carried out using a fluidized bed granulator.

10. The process according to any one of claims 1 to 9, additionally comprising a drying step.

11. The process according to any one of claims 7, 9 or 10 in which the granulation and drying steps are combined by using a fluidized bed granulator.

12. The process according to any one of claims 1 to 11, further comprising a compression step.

13. The process according to claim 12, in which said compression step is preceded by a step in which additives are added and blended.

14. Process according to any of claim 1 to 13, comprising the steps of:
(i) mixing, for a sufficient period of time, metoprolol active ingredient and, optionally, one of several additives, with an aqueous solution; then
(ii) adding PEO and mixing for a sufficient period of time;
(iii) granulating the mixture obtained from step (ii);
(iv) drying granules thus formed for a sufficient period of time;
(v) optionally, adding one or several additives, and mixing in the dry state for a sufficient period of time;
(vi) compressing the mixture obtained from the preceding step into a tablet.

15. The process according to claim 14, in which step (i) of mixing with the aqueous solution is carried out in the presence of excess liquid.

16. The process according to claim 14 or 15, in which the granulation and drying steps are combined by using a fluidized bed granulator.

17. Process according to any of claim 1 to 13, comprising the steps of:
(i) mixing, for a sufficient period of time, active ingredient and, optionally, one of several additives, with said aqueous solution; then
(ii) granulating the mixture obtained from step (i);
(iii) optionally drying the granules thus formed for a sufficient period of time;
(iv) adding PEO and optionally one or several additives, and mixing for a sufficient period of time;
(v) compressing the mixture obtained from the preceding step into a tablet.

18. The process according to claim 17, in which the mixing, granulation and drying steps are combined by using a fluidized bed granulator.

19. The process according to any one of claims 1 to 18, in which said aqueous solution consists of water, and, optionally, water-soluble additives.

20. The process according to claim 19, in which said aqueous solution comprises water and a binder in an amount up to 20%.

## Patentansprüche

1. Verfahren zur Herstellung einer Feststoffzusammensetzung, umfassend, bezogen auf das Gewicht basierend auf dem Gesamtgewicht der Zusammensetzung:
(a) von 1 bis 70% Metoprolol-Wirkstoff, welcher nicht in einer amorphen Form vorliegt,
(b) von 10 bis 95% Polyethylenoxid (PEO),
(c) wobei der Rest aus herkömmlichen Zusatzmitteln besteht, umfassend die Schritte:
(i) das Mischen des Metoprolol-Wirkstoffes und gegebenenfalls eines oder mehrerer Zusatzmittel mit einer wäßrigen Lösung für eine ausreichende Zeitdauer, dann
(ii) das Zugeben des PEO und das Mischen für eine ausreichende Zeitdauer.

2. Verfahren nach Anspruch 1, wobei der Metoprolol-Wirkstoff Metoprololtartrat ist.

3. Verfahren nach Anspruch 1 oder 2, wobei die Zusammensetzung umfaßt:
(a) von 5 bis 45% Metoprolol-Wirkstoff, welcher nicht in einer amorphen Form vorliegt,
(b) von 20 bis 70% Polyethylenoxid,
(c) wobei der Rest aus herkömmlichen Zusatzmitteln besteht, mit Ausnahme basischer Komponenten.

4. Verfahren nach einem der Ansprüche 1 bis 3, wobei das Polyethylenoxid ein Molekulargewicht aufweist, welches von 50.000 bis 8.000.000 und vorzugsweise von 300.000 bis 3.000.000 variiert.

5. Verfahren nach einem der Ansprüche 1 bis 4, wobei der Rest, welcher aus herkömmlichen Zusatzmitteln besteht, mikrokristallinen Zellstoff, Laktose, Ascorbinsäure, Pigmente, Weichmacher und Gleitmittel umfaßt.

6. Verfahren nach einem der Ansprüche 1 bis 5, worin der Schritt (i) des Mischens mit der wäßrigen Lösung in der Gegenwart von Flüssigkeitsüberschuß durchgeführt wird.

7. Verfahren nach einem der Ansprüche 1 bis 6, welches weiter einen Granulationsschritt umfaßt.

8. Verfahren nach Anspruch 7, worin der Granulationsschritt auf einem Sieb von geeigneter Maschengröße durchgeführt wird.

9. Verfahren nach Anspruch 7, worin der Granulationsschritt unter Verwendung einer Fließbettgranuliermaschine durchgeführt wird.

10. Verfahren nach einem der Ansprüche 1 bis 9, welches zusätzlich einen Trokkenschritt umfaßt.

11. Verfahren nach einem der Ansprüche 7, 9 oder 10, worin die Granulations- und Trockenschritte unter Verwendung einer Fließbettgranuliermaschine kombiniert werden.

12. Verfahren nach einem der Ansprüche 1 bis 11, welches weiter einen Verdichtungsschritt umfaßt.

13. Verfahren nach Anspruch 12, worin dem Verdichtungsschritt ein Schritt, in welchem die Zusatzmittel zugegeben und gemischt werden, vorangeht.

14. Verfahren nach einem der Ansprüche 1 bis 13, welches die Schritte umfaßt:
(i) das Mischen von Metoprolol-Wirkstoff und gegebenenfalls einem von mehreren Zusatzmitteln mit einer wäßrigen Lösung für eine ausreichende Zeitdauer, dann
(ii) das Zugeben von PEO und das Mischen für eine ausreichende Zeitdauer,
(iii) das Granulieren des in Schritt (ii) erhaltenen Gemisches,
(iv) das Trocknen der so gebildeten Körnchen für eine ausreichende Zeitdauer,
(v) gegebenenfalls das Zugeben eines oder mehrerer Zusatzmittel und das Mischen im Trockenzustand für eine ausreichende Zeitdauer,
(vi) das Verpressen des im vorherigen Schritt erhaltenen Gemisches in eine Tablette.

15. Verfahren nach Anspruch 14, worin der Schritt (i) des Mischens mit der wäßrigen Lösung in der Gegenwart eines Flüssigkeitsüberschusses durchgeführt wird.

16. Verfahren nach Anspruch 14 oder 15, worin die Granulations- und Trockenschritte unter Verwendung einer Fließbettgranuliermaschine kombiniert werden.

17. Verfahren nach einem der Ansprüche 1 bis 13, welches die Schritte umfaßt:
(i) das Mischen von Wirkstoff und gegebenenfalls einem von mehreren Zusatzmitteln mit der wäßrigen Lösung für eine ausreichende Zeitdauer, dann
(ii) das Granulieren des in Schritt (i) erhaltenen Gemisches,
(iii) gegebenenfalls das Trocknen der so gebildeten Körnchen für eine ausreichende Zeitdauer,
(iv) das Zugeben von PEO und gegebenenfalls einem oder mehreren Zusatzmitteln und das Mischen für eine ausreichende Zeitdauer,
(v) das Verpressen des im vorherigen Schritt erhaltenen Gemisches in eine Tablette.

18. Verfahren nach Anspruch 17, worin die Misch-, Granulations- und Trockenschritte unter Verwendung einer Fließbettgranuliermaschine kombiniert werden.

19. Verfahren nach einem der Ansprüche 1 bis 18, wobei die wäßrige Lösung aus Wasser und gegebenenfalls wasserlöslichen Zusatzmitteln besteht.

20. Verfahren nach Anspruch 19, wobei die wäßrige Lösung Wasser und ein Bindemittel in einer Menge bis zu 20% umfaßt.

## Revendications

1. Procédé pour préparer une composition solide comprenant en poids exprimé par rapport au poids total de la composition :
(a) de 1 à 70 % d'un ingrédient actif à base de métoprolol qui n'est pas sous forme amorphe ;
(b) de 10 à 95 % d'un oxyde de polyéthylène (PEO)
(c) le reste étant formé d'additifs classiques;
comprenant les étapes de :
(i) mélange dudit ingrédient actif à base de métoprolol et éventuellement d'un ou plusieurs additifs avec une solution aqueuse pendant une période de temps suffisante ;puis
(ii) addition du PEO et mélange pendant une durée de temps suffisante.

2. Procédé selon la revendication 1, dans lequel l'ingrédient actif à base de métoprolol est le tartrate de métoprolol.

3. Le procédé selon la revendication 1 ou 2, dans lequel ladite composition comprend :
(a) de 5 à 45 % d'ingrédient actif à base de métoprolol qui n'est pas sous forme amorphe ;
(b) de 20 à 70 % d'oxyde de polyéthylène ;
(c) le reste étant formé d'additifs classiques, à l'exclusion de constituants basiques.

4. Le procédé selon l'une quelconque des revendications 1 à 3, dans lequel l'oxyde de polyéthylène présente un poids moléculaire qui varie de 50 000 et 8 000 000 et, de préférence de 300 000 et 3 000 000.

5. Le procédé selon l'une quelconque des revendications 1 à 4, dans lequel le reste étant essentiellement formé d'additifs classiques comprenant la cellulose micro-cristalline, le lactose, l'acide ascorbique, des pigments, des agents plastifiants et des lubrifiants.

6. Le procédé selon l'une quelconque des revendications 1 à 5, dans lequel l'étape (i) de mélange avec la solution aqueuse est mise en oeuvre en présence d'un liquide en excès.

7. Le procédé selon l'une quelconque des revendications 1 à 4, comprenant en outre une étape de granulation.

8. Le procédé selon la revendication 7, dans lequel ladite étape de granulation est mise en oeuvre sur une grille ayant une dimension en mesh convenable.

9. Le procédé selon la revendication 7 dans laquelle ladite étape de granulation est effectuée à l'aide d'un granulateur à lit fluidisé.

10. Le procédé selon l'une quelconque des revendications 1 à 9, comprenant en outre une étape de séchage.

11. Le procédé selon l'une quelconque des revendications 7, 9 ou 10, dans lequel les étapes de granulation et de séchage sont combinées par emploi d'un granulateur à lit fluidisé.

12. Le procédé selon l'une quelconque des revendications 1 à 11, comprenant en outre une étape de compression.

13. Le procédé selon la revendication 12, dans lequel ladite étape de compression, est précédée d'une étape dans laquelle les additifs sont ajoutés et mélangés.

14. Le procédé selon l'une quelconque des revendications 1 à 13, comprenant les étapes de :
(i) mélange, pour une période de temps suffisante, d'un ingrédient actif à base de métoprolol et, éventuellement, de un ou plusieurs additifs avec une solution aqueuse ; puis
(ii) addition de PEO et mélange pendant une période de temps suffisante;
(iii) granulation du mélange obtenu dans l'étape (ii) ;
(iv) séchage des granulés ainsi formés pendant une période de temps suffisante ;
(v) éventuellement addition d'un ou plusieurs additifs et mélange à l'état sec pendant une période de temps suffisante ;
(vi) compression du mélange obtenu dans l'étape précédente pour le mettre sous forme de comprimés.

15. Le procédé selon la revendication 14, dans lequel l'étape (i) de mélange avec la solution aqueuse est effectuée en présence d'un liquide en excès.

16. Le procédé selon la revendication 14 ou 15, dans lequel les étapes de granulation et de séchage sont combinées par emploi d'un granulateur à lit fluidisé.

17. Le procédé selon l'une quelconque des revendications 1 à 13, comprenant les étapes de :
(i) mélange, pour une période de temps suffisante, d'un ingrédient actif à base de métoprolol et, éventuellement, de un ou plusieurs additifs avec une solution aqueuse ; puis
(ii) granulation du mélange obtenu dans l'étape (i);
(iii) éventuellement séchage des granulés ainsi formés pendant une période de temps suffisante ;
(iv) addition de PEO et éventuellement d'un ou plusieurs additifs et mélange à l'état sec pendant une période de temps suffisante ;
(v) compression du mélange obtenu dans l'étape précédente pour le mettre sous forme de comprimés.

18. Le procédé selon la revendication 17, dans lequel les étapes de mélange, de granulation et de séchage sont combinées par emploi d'un granulateur à lit fluidisé.

19. Le procédé selon l'une quelconque des revendications 1 à 18, dans lequel ladite solution aqueuse est formée d'eau et, éventuellement d'additifs hydrosolubles.

20. Le procédé selon la revendication 19, dans lequel ladite solution aqueuse comprend de l'eau et un liant dont la quantité peut atteindre 20 %.
